Europäisches Patentamt

European Patent Office

Office européen des brevets

(11) Veröffentlichungsnummer: **0 215 452**
**A2**

(12) # EUROPÄISCHE PATENTANMELDUNG

(21) Anmeldenummer: **86112627.4**

(22) Anmeldetag: **12.09.86**

(51) Int. Cl.⁴: **G01N 21/64** , **G01N 33/10** , **H04N 7/18** , **B07C 5/342**

(30) Priorität: **19.09.85 US 777886**

(43) Veröffentlichungstag der Anmeldung:
**25.03.87 Patentblatt 87/13**

(84) Benannte Vertragsstaaten:
**AT BE DE FR GB IT SE**

(71) Anmelder: **DEERE & COMPANY**
**1 John Deere Road**
**Moline Illinois 61265(US)**

(72) Erfinder: **Brizgis, Lawrence Joseph**
**4714 - 20th Avenue**
**Moline Illinois 61265(US)**
Erfinder: **Keleher, Daniel Bradley**
**903 Brown Street**
**Bettendorf Iowa 52722(US)**
Erfinder: **Bandelow, Vernon Delbert**
**Rural Route No. 3 Box 15B**
**Cambridge Illinois 61238(US)**

(74) Vertreter: **Sartorius, Peter et al**
**DEERE & COMPANY European Office, Patent**
**Department Postfach 503 Steubenstrasse**
**36-42**
**D-6800 Mannheim 1(DE)**

(54) **Verfahren zur Ermittlung der Kornqualität.**

(57) Die Vorrichtung zur Erfassung der Kornqualität ist mit einem antreibbaren Mischbehälter (14) ausgestattet, der zur Aufnahme von Kornproben dient, die mittels einer elektromagnetische Strahlen aussendenden Lampe (30) beleuchtet werden. Die von der Lampe ausgesandten Strahlen sind so bestimmt, daß beschädigtes Korn von unbeschädigtem Korn unterschieden werden kann. Eine Videokamera nimmt die reflektierten Strahlen auf und führt sie einem Computer zur Auswertung und Ermittlung des Kornbruches zu.

FIG. 1

0 215 452

## Verfahren zur Ermittlung der Kornqualität

Die Erfindung bezieht sich auf ein Verfahren zur Ermittlung der Kornqualität, insbesondere des Kornbruches von Getreide, mittels eines Computers zur Erfassung der Meßdaten, dem eine Lampe zur Beleuchtung einer Kornprobe zugeordnet ist, die elektromagnetische Strahlen auf die Kornprobe abgibt, die über den Computer ausgewertet werden.

Es ist bereits eine Vorrichtung zur Ermittlung des Kornbruches bekannt, zu der ein computergesteuertes Analysegerät mit einer Quartzlampe gehört, die infrarote Strahlungen über eine Linse auf die Oberfläche einer Kornprobe leitet, die zu analysieren ist (US-A-3 828 173). Die von der Lampe ausgehenden Strahlungen werden durch bestimmte Filter geleitet, so daß nur eine bestimmte Frequenz der Strahlungen auf die Erntegutprobe auftrifft. Die von dem Erntegut reflektierten Strahlungen sind dann zu analysieren. Von einer zugehörigen Photozelle werden entsprechende Informationen ausgesandt, die einen Aufschluß über das zu analysierende Erntegut geben. Gleichzeitig wird ein Referenzsignal durch den Filter geleitet, das als Bezugsgröße verwendet werden kann. Diese Vorrichtung dient hauptsächlich zur Bestimmung des Öl-und Proteinanteiles sowie des Feuchtigkeitsgehaltes. Mit einer derartigen Vorrichtung kann jedoch der Kornbruch nicht ermittelt werden.

Demgegenüber liegt der Erfindung die Aufgabe zugrunde, ein Verfahren zur Ermittlung der Kornqualität, insbesondere des Kornbruches, derart auszubilden und anzuordnen, daß eine genaue Erfassung über die Beschädigung des Kornes möglich ist. Diese Aufgabe ist durch die im kennzeichnenden Teil des Anspruches 1 aufgeführten Merkmale gelöst. Nach dem verwendeten Verfahren ist es möglich, auf einfache Weise eine Analyse des beschädigten Kornes durchzuführen, indem die Abbildinformationen über einen zugehörigen Computer bzw. Prozessor ausgewertet werden. Hierzu ist es vorteilhaft, daß dem Computer ein Mischgerät zugeordnet ist, das die aufgenommenen Gutproben entsprechend mischt, um sie dann anschließend zu analysieren. Die hierzu verwendete Lampe sendet ultraviolette Strahlen aus, die insbesondere den Stärkeanteil des beschädigten Erntegutes fluoreszieren und somit ein visuelles Kontrastbild zwischen dem beschädigten und nicht beschädigten Erntegut herstellen. Eine zugehörige Videokamera erzeugt die entsprechenden Abbilder bzw. Bildinformationen von den Erntegutproben und sendet das hierdurch gebildete Videosignal einem Computer zu, der die Signale digitalisiert und entsprechende Bildpunkte produziert. Ein jeder Bildpunkt entspricht der Intensität des entsprechenden von der Videokamera erstellten Abbildes. Der Computer ermittelt dann in vorteilhafter Weise den Prozentsatz der Bildpunkte, die einen Wert repräsentieren, der über der vorherbestimmbaren Schwelle liegt. Danach läßt sich der hieraus ermittelte Prozentsatz in eine Beziehung zu der sichtbar gemachten Stärke setzen und somit der Prozentsatz des beschädigten Erntegutes ermitteln. Der Computer analysiert in vorteilhafter Weise die zahlreichen Abbilder bzw. Bildinformationen von einer jeden Gutprobe und steuert automatisch das Mischgerät, so daß eine jede Gutprobe entsprechend gemischt wird, bevor eine Bildinformation analysiert wird.

Weitere vorteilhafte Ausgestaltungen der Erfindung gehen aus den Unteransprüchen hervor.

Im folgenden wird die Erfindung anhand von lediglich einen Ausführungsweg darstellenden Zeichnungen näher erläutert. Es zeigt:

Fig. 1 ein schematisches Diagramm eines Kornbruchanalysegerätes,

Fig. 2 eine Seitenansicht eines Mischgerätes,

Fig. 3 die Frontansicht des Mischgerätes gemäß Fig. 2,

Fig. 4 einen elektrischen Stromlaufplan des Steuergerätes für das Mischgerät,

Fig. 5 den Ablaufplan des Hauptprogrammes,

Fig. 6 einen weiteren Ablaufplan eines Unterprogrammes.

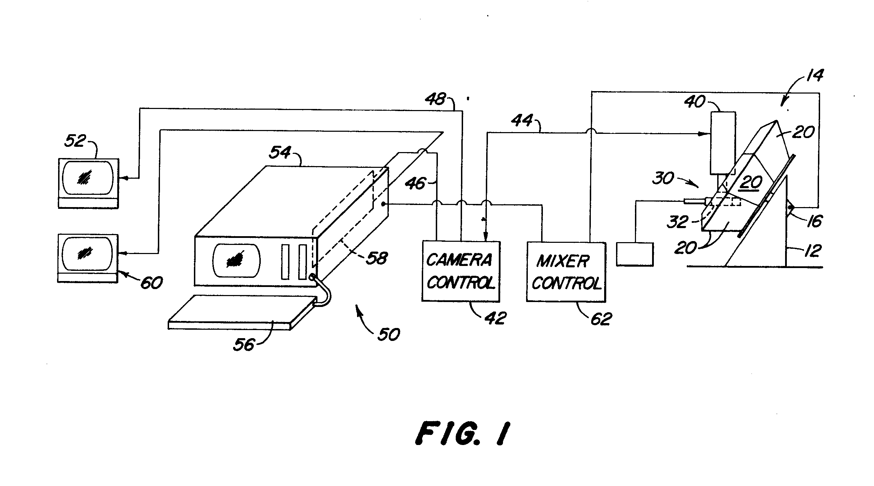

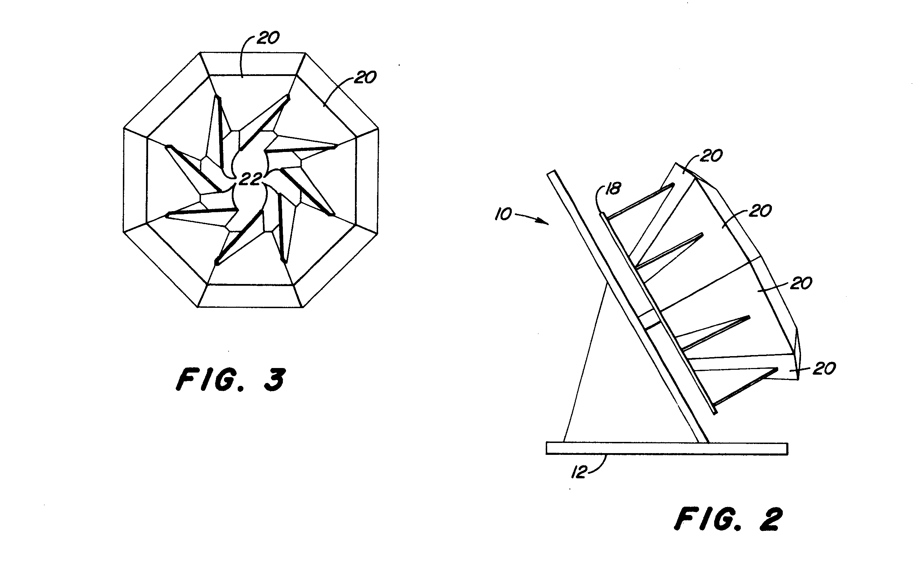

In der Zeichnung ist mit 10 ein Mischgerät eines Analysegerätes zur Feststellung von Kornbruch dargestellt, das zur Aufnahme von Kornproben dient. Das Mischgerät 10 weist eine Grundplatte 12 auf, die zur Aufnahme eines drehbar gelagerten Mischbehälters 14 dient, dessen Drehachse mit Bezug auf die horizontal verlaufende Ebene einen Winkel von 30° einschließt. Der Mischbehälter lagert auf einer Welle, die mittels eines 12 Volt-Gleichstrommotors 16 antreibbar ist. Der Mischbehälter 14 ist mit einer flachen Scheibe 18 ausgestattet, auf der zahlreiche trapezförmig ausgebildete Seitenwände 20 angeordnet sind, die den Mischbehälter 14 bilden. Der Mischbehälter ist so ausgerichtet, daß im Arbeitseinsatz die unterste Seitenwand 20 stets in einer horizontal verlaufenden Ebene verläuft. Ferner ist das Mischgerät 10 mit zahlreichen dreieckförmigen Schaufelelementen 22 ausgerüstet, die auf dem Boden 18 angeordnet sind und sich radial nach außen in Richtung der Seitenwände 20 erstrecken, wobei die äußeren Enden der Schaufelelemente 22 an der Nahtstelle zwischen zwei benachbarten Seitenwänden 20 enden. Wird beispielsweise eine Kornprobe in den Mischbehälter 14 eingegeben, so rühren die Schaufelelemente 22 die Kornprobe um, wenn der Mischbehälter angetrieben wird und sich dreht, wobei ein weiteres Paneel die unterste Stellung gemäß Fig. 2 errecht. Wie nachstehend weiter erläutert, ist der innere Teil des Mischbehälters 14 mit einem Überzug versehen, der sichtbares bzw. ultraviolettes Licht nicht reflektiert und der nicht fluoresziert. Eine Strahlungsquelle bzw. eine Lampe 30 ist derart angeordnet (siehe Fig. 1), daß die Kornprobe, die auf der unteren Seitenwand 20 aufliegt, beleuchtet wird. Die Lampe 30 weist eine Ringform mit einer mittleren Durchlaßöffnung 32 auf. Die Lampe 30 kann als ultraviolette Lampe ausgebildet sein bzw. eine Mic-O-Lite , FUV-36 Lampe sein (Astro Grid Lamp Products, Inc.). Diese Lampe weist eine eigene Stromversorgung 31 auf. Die Lampe 30 produziert langwellige, ultraviolette Strahlungen mit einer Wellenlänge von 3 650 Angström. Es wird angenommen, daß die Strahlen lediglich die stärkehaltigen Teile des Bruchkornes aus der Kornprobe fluoreszieren bzw. lumineszeren. D. h. der Stärketeil des Bruchkornes absorbiert ultraviolette Strahlungen und erzeugt dann sichtbares Licht. Es wird ferner angenommen, daß die Strahlungen den Kontrast zwischen beschädigtem Erntegut und nicht beschädigtem Erntegut sichtbar machen.

Das von der Kornprobe ausgesandte Licht in dem Mischgerät 10 wird durch eine Videokamera 40 aufgefangen, die derart angeordnet ist, daß ihr Sichtfeld durch die Öffnung 32 der Lampe fällt und somit eine Gutprobe auf der unteren Wand 20 des Mischbehälters 14 einfängt. Die Videokamera 40 ist mit einer Steuereinheit 42 verbunden. Die Steuereinheit 42 weist Steuerteile für die entfernt angeordnete Videokamera 40 auf, die ein Videosignal über ein Kabel 44 erhält und die ein Videosignal über koaxial geschützte Kabel 46 und 48 an einen Computer 50 und an einen Videomonitor 52 weitergibt. Eine geeignete Kamera und Steuereinheit stellt die 67M TV Kamera von DAGE-MTI, Inc. dar. Die Kamera kann mit einer Super Chalnicon R Röhre mit einer 1,4/25 mm Linse ausgerüstet sein. Es ist natürlich auch möglich, anders ausgerüstete Kameras zu verwenden. Im Arbeitseinsatz sind die Videokamera 40 und das Mischgerät vorzugsweise mit einer lichtundurchlässigen Abdeckung versehen, so daß die Kornprobe sowie die Kamera nicht Licht ausgesetzt sind.

Der Computer 50 kann beispielsweise von Sanyo MBC 775/ tragbar 256k sein und mit einem Diskettenlaufwerk, einem Farbbildschirm und einer Tastatur 56 ausgestattet sein. Ferner ist in dem Computer 50 ein Digitalisierungssystem 58 (interface card) eingebaut.

Ein geeignetes Digitalisierungssystem ist beispielsweise das PC-EYE (TM) Serie 1 000 Video Capture System. Dieses System enthält eine Interface Karte 58 und benötigt eine Software auf einer Floppy Diskette (nicht dargestellt). Die Interface Karte empfängt ein analoges Videosignal über das Kabel 46 und zeigt 640 Zeichen auf 200 Zeilen von 6-Bit Pixels (64er Grauton). Die Signale, die die Pixelreihe repräsentieren, kommunizieren mit dem Computer 50 und einem weiteren Videobildschirm 60. Beide Videobildschirme 52 und 60 können beispielsweise Hitachi VM-910A Bildschirme sein (schwarz-weiß, 28 cm).

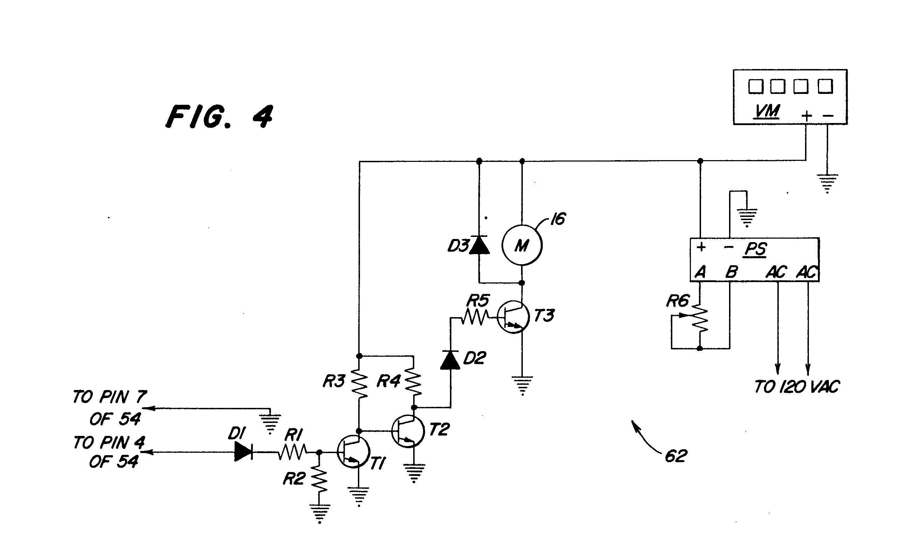

Ein Standardgerät RS 232 des Computers 54 ist mit einem Misch-Steuer-Stromkreis 62 verbunden (siehe Fig. 4). Der Misch-Steuer-Stromkreis 62 reagiert auf ein binäres Ein-und Aus-Signal, das durch den Computer 54 erzeugt wird, indem der Stromfluß durch den Gleichstrommotor 16 erfaßt wird. Der Ausgang des Misch-Steuer-Stromkreises 62 kann zwischen 0 und 12 Volt eingestellt werden. Vorzugsweise wird er auf 9 Volt eingestellt. Ist das Signal des Computers 54 klein, so fließt kein Strom durch den Gleichstrommotor 16. st das Computer-Ausgangssignal hoch, ist der Transistor T3 leitend und Strom fließt durch den Gleichstrommotor 16.

Der Misch-Steuer-Stromkreis 62 enthält folgende Komponenten (es sind jedoch andere Komponenten mit anderen Abmessungen möglich):

| R1 | Widerstand | 5,11 k Ohm |
| R2 | Widerstand | 2,0 k Ohm |

| R3 | Widerstand | 7,5 k Ohm |
| R4 | Widerstand | 5,11 k Ohm |
| R5 | Widerstand | 4,75 k Ohm |
| R6 | Potentiometer | 0 - 1 000 Ohm |
| D1, D2 | Dioden | IN 914 |
| D3 | Diode | IN 4004 |
| T1, T2 | Transistoren | 2N2222A |
| T3 | Transistor | TIP122 |
| PS | Kraftversorgung | Emerson ECV 12 N 1,7 |
| VM | Voltmeter | Martel Model 3554/2 |
| | | 0 - 20 VDC |

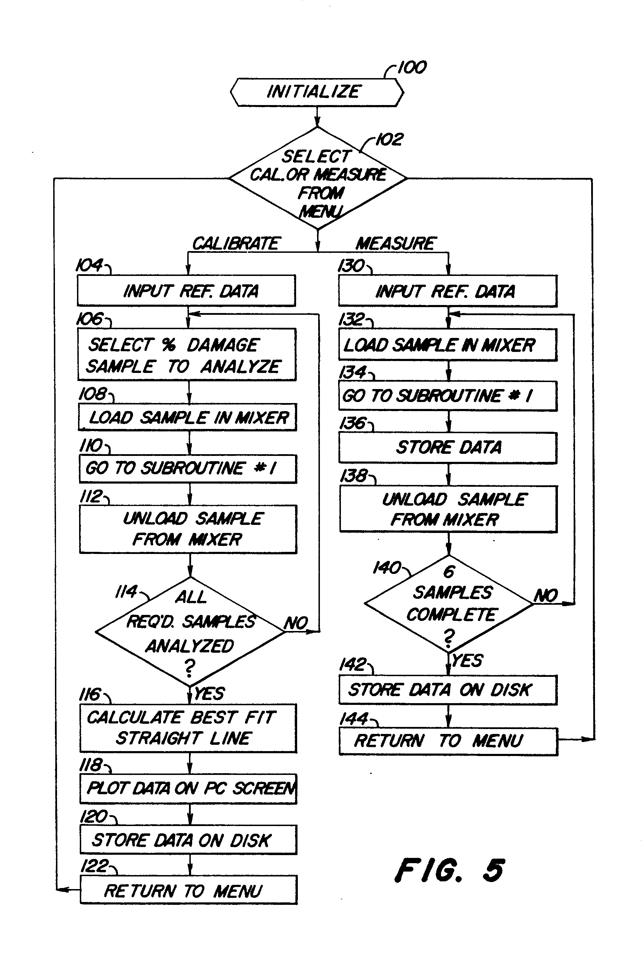

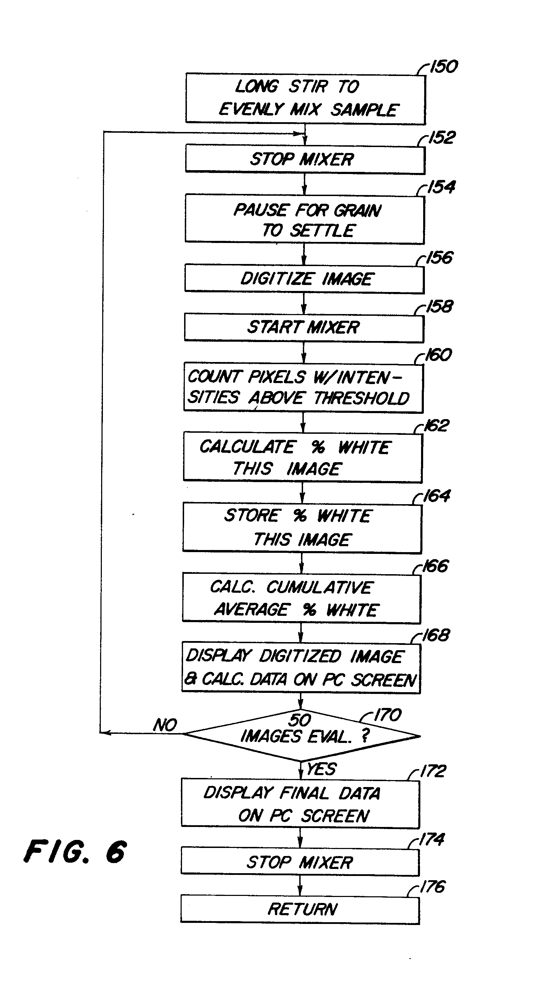

Der Computer 54 führt einen Algorithmus aus, der nachfolgend beschrieben und in den Figuren 5 und 6 veranschaulicht ist. Für weitere Details bezüglich des Algorithmus wird auf den Microfilm-Anhang verwiesen. Der Algorithmus beginnt mit der Inszenierung in Stufe 100 und geht dann in den Kalibrierungs- bzw. Meßzweig über, der durch die Stufe 102 veranschaulicht ist. Der Kalibrierungszweig beginnt bei der Stufe 104, und zwar an der Stelle, an der der Benutzer Datensicherungsinformationen eingibt. An der Stufe 106 wird der Prozentsatz des Umfanges der Beschädigung einer bekannten Kornprobe eingegeben. Beispielsweise ist das System so ausgelegt, daß zahlreiche Proben mit unterschiedlichen Beschädigungs-Prozentsätzen, beispielsweise 0, 10, 20, 30 und 40, verwendet werden können. Bei der Stufe 108 instruiert der Anwender, die ausgewählte Probe in das Mischgerät 10 einzugeben. Stufe 110 ruft ein Unterprogramm #1 auf, das nachfolgend beschrieben und in Fig. 6 veranschaulicht ist.

Das Unterprogramm #1 beginnt bei der Stufe 150, in der ein Signal erzeugt wird, das veranlaßt, daß der Misch-Steuer-Stromkreis 62 den Gleichstrommotor 16 des Mischgerätes 10 beaufschlagt, so daß die Kornprobe für eine längere Zeit zwischen 5 und 8 Sekunden herumgerührt wird. Nach dem Rührvorgang wird das Mischgerät 10 an der Stufe 152 gestoppt. Die Stufe 154 erlaubt der Kornprobe, sich für eine gewisse Zeit zu beruhigen bzw. abzusetzen, und zwar für eine Periode von ca. 3 Sekunden.

An der Stelle 156 wird das Videosignal von der Videokamera 40 digitalisiert (dieses gibt ein Bild der abgesetzten bzw. beruhigten Kornprobe wieder), und zwar aufgrund der Software, die mit der Interface Karte 58 zusammenwirkt. Hierdurch wird das Bild durch 640 Zeichen bei 200 Zeilen wiedergegeben. Jedes Bild hat eine Binärnummer (0 -63), die die Helligkeit des entsprechenden Teils des Abbildes repräsentiert.

Nachdem das Abbild digitalisiert ist, verursacht die Stufe 158, daß das Mischgerät 10 startet, so daß die Kornprobe gemischt werden kann, wobei dieses digitalisierte Abbild ebenfalls wie folgt analysiert wird.

In Stufe 160 erhält man eine Zählung der Zahl der Pixels bzw. Bilder, die eine Helligkeitsstufe haben, die eine bestimmte Schwelle überschreitet. Diese Stufe bzw. Schwelle kann durch entsprechende Einstellung "schwarz" und "weiß" auf der Interface Karte 58 eingestellt werden. Es ist beispielsweise möglich, beide Einstellungen auf denselben Wert zu bringen, und zwar zwischen 38 und 42, so daß für eine unbeschädigte Kornprobe lediglich 2 bis 4 % der Bildpunkte einen Wert erreichen, der die Schwelle überschreitet.

In der Stufe 162 wird die Zählung in einen Weiß-Wert-Prozentsatz umgewandelt, der dem Prozentsatz der Bildpunkte entspricht, die die Schwelle überschreiten. Der Weiß-Wert-Prozentsatz wird dann in der Stufe 164 gespeichert. Dann wird in der Stufe 166 ein kumulierter Durchschnitts-Weiß-Wert-Prozentsatz für alle Bilder bestimmt, die sich auf eine besondere Kornprobe beziehen.

In der Stufe 168 erscheint das digitalisierte Bild auf dem Monitor des Computers 54, und zwar mit den in den Stufen 160, 162 und 166 aufgenommenen bzw. festgelegten Daten. Wird weniger als ein bestimmter Betrag (beispielsweise 50) der Bilder erzeugt, dann bewirkt die Stufe 170 eine Rückgabe des Algorithmus an die Stufe 152, um das Mischgerät 10 zu stoppen und somit ein anderes Bild zu produzieren. Der Algorithmus bewirkt dann in der Stufe 172 die Abgabe der Abschlußdaten aus den Stufen 160, 162 und 166, die dann auf dem Bildschirm des Computers 54 wiedergegeben werden. Schließlich stoppt die Stufe 172 das Mischgerät 10, und die Stufe 174 bewirkt eine Rückkehr des Hauptprogrammes.

Wie aus Fig. 5 hervorgeht, setzt sich der Kalibrierungszweig an der Stelle bzw. Stufe 112 fort, an der das Mischgerät 10 die Kornprobe wieder abgibt. An der Stufe 114 wird der Algorithmus zurück auf die Stufe 106 geführt, um eine weitere Kornprobe entsprechend zu bearbeiten. Der Algorithmus setzt sich dann in der Stufe 116 fort.

Vorzugsweise weist eine Anzahl von Kornproben einen bekannten Bruch-Betrag auf, der in dem Kalibrierungszweig bearbeitet wird. Beispielsweise werden vorzugsweise Proben analysiert, die einen bekannten Bruch-Wert von 0, 10, 20, 30 und 40 % aufweisen.

In der Stufe 116 wird dann eine gerade Linie oder eine andere geeignete Funktion bestimmt, die am besten zwischen dem bekannten Bruch-Wert und dem gemessenen kumulierten Durchschnitts-Prozent-Weiß-Wert liegt. In der Stufe 118 wird dieses Verhältnis und die dem zugrunde liegenden Daten auf dem Bildschirm des Computers 54 veranschaulicht. In der Stufe 120 werden die Daten gespeichert, beispielsweise auf einer Speicherscheibe. In der Stufe 122 wird bewirkt, daß der Algorithmus zur Stufe 102 zurückkehrt, so daß die Bedienungsperson wieder entweder den Kalibrierungszweig oder den Meßzweig auswählen kann.

Der Meßzweig beginnt bei der Stufe 130, an der die Bedienungsperson die Datenspeicherung eingeben und sichern kann. An der Stufe 132 wird die Bedienungsperson darauf hingewiesen, die Gutprobe in dem Mischgerät 10 aufzunehmen, wobei der Kornbruch zu diesem Zeitpunkt unbekannt ist. In der Stufe 134 wird der Algorithmus auf das Unterprogramm gebracht (siehe Fig. 6, Stufen 150 bis 176).

Nachdem das Unterprogramm ausgeführt ist, kehrt der Algorithmus auf die Stufe 136 zurück, so daß die aus dem Unterprogramm hervorgehenden Daten gespeichert werden können. In der Stufe 138 wird die Bedienungsperson informiert, das Mischgerät 10 mit der Gutprobe zu entladen. Wenn weniger als 6 Gutproben analysiert worden sind, bewirkt die Stufe 140, daß der Algorithmus die Stufen 132 bis 138 wiederholt. Für einen Teil des Erntegutes mit unbekannter Bruchrate werden beispielsweise (in etwa 6) kleine Proben - (beispielsweise zwischen 8 und 12 oz. = 227 bis 340 g) aufs Geratewohl herausgenommen und dann analysiert, um einen kumulierten Durchschnitts-Weiß-Wert-Prozentsatz zu erhalten, der der Bruchrate dieser Probe entspricht. Dadurch werden Fehlermessungen vermieden, die beispielsweise dann auftreten, wenn lediglich eine einzige nicht repräsentative Probe analysiert wird. Nachdem in etwa 6 Proben analysiert worden sind, werden die analysierten Daten bei der Stufe 142 gespeichert, nachdem die Stufe 144 eine Rückkehr auf die Menüauswahlstufe 102 herbeigeführt hat.

Der kumulierte Durchschnitts-Weiß-Wert-Prozentsatz, der in den Stufen 130 bis 144 ermittelt wird, und die direkte Verhältnislinie, die in der Stufe 116 ermittelt worden ist, können verwendet werden, um den Prozentsatz des Kornbruches für eine unbekannte Probe zu ermitteln.

Die in der Beschreibung dargestellte Methode zur Ermittlung des Bruchwertes des Kornes kann auch für andere Erntegutsorten eingesetzt werden, beispielsweise für Mais, Korn, Raps oder dergleichen. Wird beispielsweise diese Meßmethode bei einem Mähdrescher eingesetzt, so ist ein Mischgerät nicht erforderlich. In diesem Fall wird beispielsweise eine Lampe oder eine Kamera in die Nähe des Kornfördersystems der Maschine gebracht. Die aus der Meßmethode gewonnenen Werte können auch als Eingangssignal für ein Realzeit-Steuersystem verwendet werden, wenn beispielsweise ein leistungsfähiger Computer eingesetzt wird.

**Ansprüche**

1. Verfahren zur Ermittlung der Kornqualität, insbesondere des Kornbruches von Getreide, mittels eines Computers zur Erfassung der Meßdaten, dem eine Lampe (30) zur Beleuchtung einer Kornprobe zugeordnet ist, die elektromagnetische Strahlen auf die Kornprobe abgibt, die über den Computer ausgewertet werden, dadurch gekennzeichnet, daß die von der Lampe (30) ausgesandten Strahlen, die auf das Erntegut bzw. Korn abgegeben und wieder reflektiert werden, eine derartige Wellenlänge aufweisen, daß unbeschädigtes Erntegut bzw. Korn von beschädigtem Erntegut bzw. Korn unterschieden wird, wobei eine Videokamera - (40) die von der Gutprobe ausgesandten Strahlen auffängt, ein Abbild produziert und ein das Abbild beinhaltendes Videosignal weiterleitet, und daß der Computer mit einem das Videosignal in zahlreiche Bildpunkte (pixels) mittels einer Digitalisierungsvorrichtung umwandelnden Prozessor (50) ausgerüstet ist, wobei ein jeder Bildpunkt [pixel] einen Wert aufweist, der dem entsprechenden Bildteil des Abbildes entspricht, wobei die Anzahl der Bildpunkte - (pixels) mittels einer Meßvorrichtung (100 -176) erfaßt wird und einen Wert aufweist, der oberhalb einer bestimmten Schwelle liegt, die so festgelegt ist, daß der Wert der Bildpunkte diese Schwelle überschreiten kann, wobei dieser Wert im wesentlichen nur die Teile des Abbildes repräsentiert, die den beschädigten Teilen des Erntegutes entsprechen, wobei dieser ermittelte Wert die Angabe über den Kornbruch bzw. die Erntegutqualität darstellt.

2. Verfahren nach Anspruch 1, dadurch gekennzeichnet, daß die von der Lampe (30) ausgehenden und die Erntegutprobe beleuchtenden Strahlen nur den beschädigten Teil des Erntegutes fluoreszieren.

3. Verfahren nach Anspruch 2, dadurch gekennzeichnet, daß die das Erntegut bestrahlende Lampe (30) langwellige, ultraviolette Strahlen aussendet.

4. Vorrichtung zur Durchführung des Verfahrens nach Anspruch 1, dadurch gekennzeichnet, daß das Erntegut in einem Mischbehälter (14) aufgenommen ist, der auf einer Welle angeordnet und mittels eines Motors (16) derart drehbar ist, wobei das im Mischbehälter (14) aufgenommene Erntegut gemischt wird, wobei der Motor (16) auf einer Grundplatte (12) angeordnet ist und in Abhängigkeit von ihm zugeführten Steuersignalen arbeitet.

5. Vorrichtung nach Anspruch 4, dadurch gekennzeichnet, daß der Mischbehälter (14) zahlreiche im Innern des Gehäuses hochstehend angeordnete Rührwerksblätter (22) aufweist, die beim Drehen des Mischbehälters (14) das Erntegut durchmischen.

6. Verfahren nach Anspruch 4, dadurch gekennzeichnet, daß der Motor (16) zum Antrieb des Mischbehälters (14) über eine elektrische Leitung an einen Misch-Steuer-Stromkreis (62, 100, 176) zur Steuerung der Drehzahl des Mischbehälters - (14) angeschlossen ist.

7. Verfahren nach Anspruch 4, dadurch gekennzeichnet, daß der Prozessor (156) zur Verarbeitung und Weitergabe von Steuersignalen nachfolgend zahlreiche Bildinformationen von einer einzelnen Kornprobe erhält, dem eine Steuereinheit - (152, 154, 158) zugeordnet ist, wobei ein Signal zur Drehsteuerung des Mischbehälters ausgesandt wird, bevor die Bilderfassung und -verarbeitung abgeschlossen ist.

8. Vorrichtung nach Anspruch 1, dadurch gekennzeichnet, daß die Bildinformationen auf einem Bildschirm wieder gegeben werden, dem ein Prozessor (54, 168) zugeordnet ist.

9. Verfahren zur Erfassung der Erntegutqualität, insbesondere des Kornbruches, gekennzeichnet durch folgende Verfahrensschritte:

1. der Mischbehälter (14) zur Aufnahme der Erntegutproben wird über den Motor (16) in Abhängigkeit von dem Motor übermittelten Steuersignalen angetrieben, und die Gutproben werden dann durchmischt;

2. die Lampe (30) strahlt die Erntegutproben mit ultraviolettem Licht an, das den Stärkeanteil des beschädigten Kornes fluoresziert, um beschädigtes Korn von unbeschädigtem zu unterscheiden, wozu die emittierten und reflektierten elektromagnetischen Strahlen von dem Korn als Meßgröße dienen;

3. eine Videokamera (40) fängt die reflektierenden und emittierenden Strahlen von der Kornprobe auf und bildet zahlreiche Abbildinformationen, woraus ein Videosignal erstellt wird;

4. ein Abbild-Analysegerät und eine Steuereinheit (50, 62) sind einer Vorrichtung zur Digitalisierung des Videosignals zur Erstellung zahlreicher Bildpunkte (pixels) zugeordnet, wobei ein jeder Bildpunkt einen Wert darstellt, der der Intensität des zugehörigen Teils des Abbildes entspricht;

5. eine Vorrichtung (150, 158, 152, 154) erzeugt ein Motor-Steuersignal, das dafür sorgt, daß sich der Mischbehälter entsprechend dreht, um die Kornprobe derart zu mischen, daß jedes Abbild ein unterschiedliches Abbild von der Kornprobe repräsentiert.

10. Vorrichtung nach Anspruch 9, dadurch gekennzeichnet, daß dem Abbild-Analysegerät und der Steuereinheit (50, 62) eine weitere Vorrichtung (166) zugeordnet ist, die einen kumulierten Durchschnittswert von den zahlreichen Abbildern erstellt.

FIG. 1

0 215 452

FIG. 3

FIG. 2

0 215 452

# FIG. 4

VM + −

D3 M 16

R5 T3

R6 PS
+ − PS
A B AC AC

R3 R4 D2

TO PIN 7
OF 54

TO PIN 4
OF 54

DI R1 T1
R2 T2

62

TO 120 VAC

0 215 452

FIG. 5

0 215 452

```
                              ┌──────────────────────┐  ╭150
                              │   LONG STIR TO       │
                              │ EVENLY MIX SAMPLE    │
                              └──────────┬───────────┘
                                         ▼
                              ┌──────────────────────┐  ╭152
                              │     STOP MIXER       │
                              └──────────┬───────────┘
                                         ▼
                              ┌──────────────────────┐  ╭154
                              │   PAUSE FOR GRAIN     │
                              │     TO SETTLE         │
                              └──────────┬───────────┘
                                         ▼
                              ┌──────────────────────┐  ╭156
                              │   DIGITIZE IMAGE      │
                              └──────────┬───────────┘
                                         ▼
                              ┌──────────────────────┐  ╭158
                              │     START MIXER       │
                              └──────────┬───────────┘
                                         ▼
                              ┌──────────────────────┐  ╭160
                              │ COUNT PIXELS W/INTEN- │
                              │ SITIES ABOVE THRESHOLD│
                              └──────────┬───────────┘
                                         ▼
                              ┌──────────────────────┐  ╭162
                              │  CALCULATE % WHITE    │
                              │    THIS IMAGE         │
                              └──────────┬───────────┘
                                         ▼
                              ┌──────────────────────┐  ╭164
                              │   STORE % WHITE       │
                              │    THIS IMAGE         │
                              └──────────┬───────────┘
                                         ▼
                              ┌──────────────────────┐  ╭166
                              │  CALC. CUMULATIVE     │
                              │  AVERAGE % WHITE      │
                              └──────────┬───────────┘
                                         ▼
                              ┌──────────────────────┐  ╭168
                              │ DISPLAY DIGITIZED IMAGE│
                              │ & CALC. DATA ON PC SCREEN│
                              └──────────┬───────────┘
                                         ▼
                    NO               ╱  50  ╲            ╭170
               ◄─────────────────────  IMAGES EVAL. ?
                                     ╲        ╱
                                         │ YES
                                         ▼
                              ┌──────────────────────┐  ╭172
                              │  DISPLAY FINAL DATA   │
                              │    ON PC SCREEN       │
                              └──────────┬───────────┘
                                         ▼
                              ┌──────────────────────┐  ╭174
                              │     STOP MIXER        │
                              └──────────┬───────────┘
                                         ▼
                              ┌──────────────────────┐  ╭176
                              │      RETURN           │
                              └──────────────────────┘
```

**FIG. 6**